(19) Europäisches Patentamt / European Patent Office / Office européen des brevets

(11) **EP 2 810 690 A1**

(12) **EUROPEAN PATENT APPLICATION**

(43) Date of publication:
**10.12.2014 Bulletin 2014/50**

(51) Int Cl.:
**A61N 1/36** (2006.01)     A61N 1/372 (2006.01)
**G06F 19/00** (2011.01)

(21) Application number: **13170846.3**

(22) Date of filing: **06.06.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Sapiens Steering Brain Stimulation
B.V.
5656 AE  Eindhoven (NL)**

(72) Inventors:
• **Åström, Mattias
  5509 KD Veldhoven (NL)**
• **Martens, Hubert Cécile François
  5611 ND Eindhoven (NL)**

(74) Representative: **Prinz & Partner mbB
Patentanwälte
Rundfunkplatz 2
80335 München (DE)**

(54)    **A system for planning and/or providing a therapy for neural applications**

(57)    The present invention relates to a system for planning and/or providing a therapy for neural applications, especially for neurostimulation and/or neurorecording applications, comprising at least one lead (300), the lead having a plurality of electrodes (132), further comprising a processing means (400) being capable to calculate and/or to determine and/or to process characterizing data of a stimulation field (F) being provided by the electrodes (132), the characterizing data of the stimulation field (F) comprising at least two different field components (FC1; FC2), wherein the at least two different field components are a first field component (FC1) having a first field vector and a second field component (FC2) having a second field vector having a second field vector different from the first field vector, wherein the processing means (400) is further configured such that the directional component of the stimulation field (F) may be determined based on the characterizing data of the stimulation field (F). Furthermore, the present invention relates to a system for neural applications (100) and to a method for determining the directional component of a stimulation field (F) provided by the electrodes (132) of a lead (300) based on characterizing data of the stimulation field (F).

FIG. 4

EP 2 810 690 A1

**Description**

The present invention relates to a system for planning and/or providing a therapy for neural applications, especially a neurostimulation and/or neurorecording applications and to system for neural applications and a method for determining the directional component of the stimulation field.

Implantable neurostimulation devices have been used for the past ten years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the probes are connected to an implantable current pulse generator.

Currently, systems are under development with more, smaller electrodes in a technology based on thin film manufacturing. These novel systems consist of a lead made from a thin film based on thin film technology, as e.g. described in WO 2010/055453 A1. The thin film leads are fixed on a stylet material to form a probe. These probes will have multiple electrode areas and will enhance the precision to address the appropriate target in the brain and relax the specification of positioning. Meanwhile, undesired side effects due to undesired stimulation of neighbouring areas can be minimized.

Leads that are based on thin film manufacturing are e.g. described by US 7,941,202 and have been used in research products in animal studies.

In existing systems, the DBS lead has e.g. four 1.5 mm-wide cylindrical electrodes at the distal end spaced by 0.5 mm or 1.5 mm. The diameter of the lead is 1.27 mm and the metal used for the electrodes and the interconnect wires is an alloy of platinum and iridium. The coiled interconnect wires are insulated individually by fluoropolymer coating and protected in an 80A urethane tubing. With such electrode design, the current distribution emanates uniformly around the circumference of the electrode, which leads to stimulation of all areas surrounding the electrode.

The lack of fine spatial control over field distributions implies that stimulation easily spreads into adjacent structures inducing adverse side-effects in about 30% of the patients. To overcome this problem, systems with high density electrode arrays are being developed, hence providing the ability to steer the stimulation field to the appropriate target.

The clinical benefit of DBS is largely dependent on the spatial distribution of the stimulation field in relation to brain anatomy. To maximize therapeutic benefits while avoiding unwanted side-effects, precise control over the stimulation field is essential.

During stimulation with existing DBS leads there is an option to use monopolar, bipolar, or even tripolar stimulation. Neurostimulator devices with steering brain stimulation capabilities can have a large number of electrode contacts (n > 10) to which electrical settings such as current sources or grounding can be applied. Stimulation may be considered monopolar when the distance between the anode and cathode is several times larger than the distance of the cathode to the stimulation target. During monopolar stimulation in homogeneous tissue the electric field is distributed roughly spherical similar to the field from a point source. When the anode is located close to the cathode the distribution of the field becomes more directed in the anode-cathode direction. As a result the field gets denser and neurons are more likely to be activated in this area due to a higher field gradient.

The mechanisms of DBS are unknown. However, it is hypothesized that polarization (de- and/or hyperpolarization) of neural tissue is likely to play a prominent role for both suppression of clinical symptoms, as well as induction of stimulation-induced side-effects. In order to activate a neuron it has to be depolarized. Neurons are depolarized more easily close to the cathode than by the anode (about 3-7 times more depending on type of neuron, etc.).

Therefore, compared to monopolar stimulation the effect of bipolar stimulation is less spread of the electric field, a denser electric field between the anode and cathode, and more activated neurons close to the cathode. Bipolar stimulation is therefore used to focus the field to certain areas in cases when beneficial stimulation is not obtained during monopolar stimulation.

During image guided programming of the DBS device the stimulation field may be visualize to the user in various ways. Commonly, visualization techniques such as 2D contours, 3D isosurfaces, or colour-coded field maps, are used to display the distribution of the stimulation field. However, the directional component of the stimulation field, which is relevant from a neural activation point of view, is not determined and thus also not presented.

It is therefore an object of the present invention, to improve a system for planning and/or providing a therapy for neural applications, especially a system for neurostimulation and/or neurorecording applications, and system for neural applications and a method for determining the directional component of the stimulation field, in particular in that the directional component of the stimulation field, which is relevant from a neural activation point of view, is determined and thus can be presented and visualized.

The above object is solved according to the present invention by a system for planning and/or providing a therapy for neural applications according to claim 1. Accordingly, a system for planning and/or providing a therapy for neural applications is provided, especially for neurostimulation and/or neurorecording applications, comprising at least one lead, the lead having a plurality of electrodes, further comprising a processing means being capable to calculate and/or to determine and/or to process characterizing data of a stimulation field being provided by the electrodes, the characterizing data of the stimulation field comprising at least two different field components, wherein the at least two different field components are a first field component having a first field vector and a second field component having a second field vector having a second field vector different from the first field vector, wherein the processing means is further configured such that the directional component of the stimulation field may be determined based on the characterizing data of the stimulation field.

By this the advantage is achieved that the directional component of the stimulation field, which is relevant from a neural activation point of view, is determined and thus can be presented and visualized.

So, a concept and system can be provided that visualizes a stimulation field, such as an electric field, dependent on the spatial direction of the stimulation field vectors, such as the electric field vectors.

The field vector may define the direction of the electric field, i.e. e.g. the stimulation field. The direction of the electric field may be defined by the force that is exerted on a positively charged particle.

Especially, the lead may be a lead for neural applications, preferably a lead for a neurostimulation and/or neurorecording system. Such a neurostimulation and/or neurorecording system may be e.g. a DBS system.

The lead may e.g. comprise at least one thin film, whereby the thin film comprises a proximal end and a distal end, the lead further comprising a plurality of electrodes on the distal end of the thin film.

The thin film may include at least one electrically conductive layer, preferably made of a biocompatible material. The thin film may be assembled to the carrier and further processed to constitute the lead element. The thin film for a lead is preferably formed by a thin film product having a distal end, a cable with metal tracks and a proximal end. The distal end of the thin film may be forming a part of the distal end of the lead or substantially the distal end of the lead.

The distal end of the lead may be the end of the lead, which is in the implanted state of the lead the remote end of the lead with regard to the body surface area. In particular, in case of a lead for brain application, the distal end of the lead is the lower end of the lead, which is remote to the burr-hole of the skull, through which the lead is implanted.

There may be an Advanced Lead Can element, which may comprise electronic means to address the plurality of electrodes and at least one Advanced Lead Can connecting means. Further, the Advanced Lead Can element may be hermetically or substantially hermetically sealed and may comprise electronic means to address the plurality of electrodes on the distal end of the thin film, which is arranged at the distal end and next to the distal tip of the lead. The plurality of electrodes may comprise more than 5-10 electrodes, e.g. 16 or 32 electrodes or in preferred embodiments e.g. 64 electrodes or more. The electrodes may be arranged such that the electrodes are substantially evenly distributed arranged all over the distal end of the lead.

Furthermore, it is possible that the plurality of electrodes forms a complex geometrical array and/or that the plurality of electrodes is arranged circumferentially around at least one section of the lead, especially around a section next to the distal tip end of the lead.

By means of a complex array a stimulation field of any desired shape may be formed and may be adjusted according to the patient's needs. So, a suitable and accurate tailor-made stimulation field may be provided.

A complex array may be formed by a plurality of electrodes, which are arranged circumferentially around a section next to the distal tip end of the lead. The electrodes may be e.g. arranged non-planar and non-coaxial and likewise a leopard pattern and may form a regular array. Several electrodes may form at one level a ring around the lead and the next ring may be slightly displaced such that e.g. one electrode of the second ring is partially arranged within the gap between to electrodes of the first ring. So, there are rings of electrodes in radial direction of the lead and columns of electrodes in axial direction of the lead.

Furthermore it is possible that the first field component is an anodic field component and the second field component is a cathodic field component.

A cathodic field component may be provided by at least one (or more) electrode(s) through which e.g. electric current flows out. An anodic field component may be provided by at least one (or more) electrode(s) through which e.g. electric current flows in.

Additionally, it is possible that the first field component is a field component having a field vector pointing away from the lead and the second field component is a field component having a field vector pointing towards from the lead.

Moreover it is possible that the processing means is configured such that the directional component of the stimulation field is determined directly and/or indirectly by determining and/or analysing the location and the first and/or second field vector.

Furthermore, it is possible that the processing means is configured such that two components of the location and the first and/or second field vector are determined and are used for the determination of the directional component of the stimulation field, especially that only the x-component and the y-component of the location and the first and/or second field vector are determined and are used for the determination of the directional component of the stimulation field.

The vectors and also the location, which can be considered also as a vector, may comprise a x-component, a y-component and a z-component. For instance, in a case, where the longitudinal axis of the lead is located along the z-axis, only the x-component and the y-component are to be considered.

By this, the advantage is achieved that the determination process is simplified and thus accelerated. Further, the determination process requires less calculation capability of the processing means and also less storage capability of the processing means. Additionally, by this the determination problem is reduced from a 3D-problem and a 3D-calculation to a 2D-problem and a 2D-calculation.

Additionally, it is possible that the processing means is configured such that the dot product of the location and the first and/or second field vector is determined, wherein especially it is determined that the first field component is a field component having a field vector pointing away from the lead if the dot product is >0, i.e. has a value larger than zero, and the second field component is a field component having a field vector pointing towards from the lead if the dot product is <0, i.e. has a value lower than zero.

Moreover, the present invention relates to a system for neural applications with the features of claim 8. Accordingly, a system for neural applications is provided, especially a system for neurostimulation and/or neurorecording applications, for instance a deep brain stimulation system. The system for neural applications comprises at least one system for planning and/or providing a therapy for neural applications according to one of claims 1 to 7.

Furthermore, the present invention relates to a method for determining the directional component of the stimulation field with the features of claim 9. Accordingly, a method for determining the directional component of the stimulation field provided by the electrodes of a lead is provided, wherein the lead is a lead for neural applications, especially for neurostimulation and/or neurorecording applications, wherein characterizing data of the stimulation field comprising at least two different field components are calculated and/or determined and/or processed, wherein the at least two different field components are a first field component having a first field vector and a second field component having a second field vector having a second field vector different from the first field vector.

Additionally, it is possible that the plurality of electrodes forms a complex geometrical array and/or that the plurality of electrodes is arranged circumferentially around at least one section of the lead, especially around a section next to the distal tip end of the lead.

It is also possible that the first field component is an anodic field component and the second field component is a cathodic field component and/or the first field component is a field component having a field vector pointing away from the lead and the second field component is a field component having a field vector pointing towards from the lead.

Further, it is possible that the directional component of the stimulation field is determined directly and/or indirectly by determining and/or analysing the location and the first and/or second field vector.

Moreover, it is possible that two components of the location and the first and/or second field vector are determined and are used for the determination of the directional component of the stimulation field, especially that only the x-component and the y-component of the location and the first and/or second field vector are determined and are used for the determination of the directional component of the stimulation field.

Additionally, it is possible that the dot product of the location and the first and/or second field vector is determined, wherein especially it is determined that the first field component is a field component having a field vector pointing away from the lead if the dot product is >0, i.e. has a value larger than zero, and the second field component is a field component having a field vector pointing towards from the lead is <0, i.e. has a value lower than zero.

The method may be conducted with at least one system for planning and/or providing a therapy for neural applications according to one of claims 1 to 7 and/or a system for neural applications according to claim 8.

It is possible that the above described method and the preferred embodiments thereto for planning and/or providing a therapy for neural applications are only used in-vitro or for testing and planning purposes only.

However, also it is also explicitly disclosed that the above described method and the preferred embodiments thereto for planning and/or providing a therapy for neural applications may be used for planning and/or providing a therapy for neural applications, especially a neurostimulation and/or neurorecording applications like DBS when the lead is implanted into the patient during therapy.

Further details and advantages of the present invention shall be described hereinafter with respect to the drawings:

Fig. 1:     a schematical drawing of a neurostimulation system for deep brain stimulation (DBS);

Fig. 2:     a further schematical drawing of a probe of a neurostimulation system for deep brain stimulation (DBS) and its components;

Fig. 3:     a schematical drawing of a probe system according to the present invention; and

Fig. 4:     an overview of a possible embodiment of the system according to the invention.

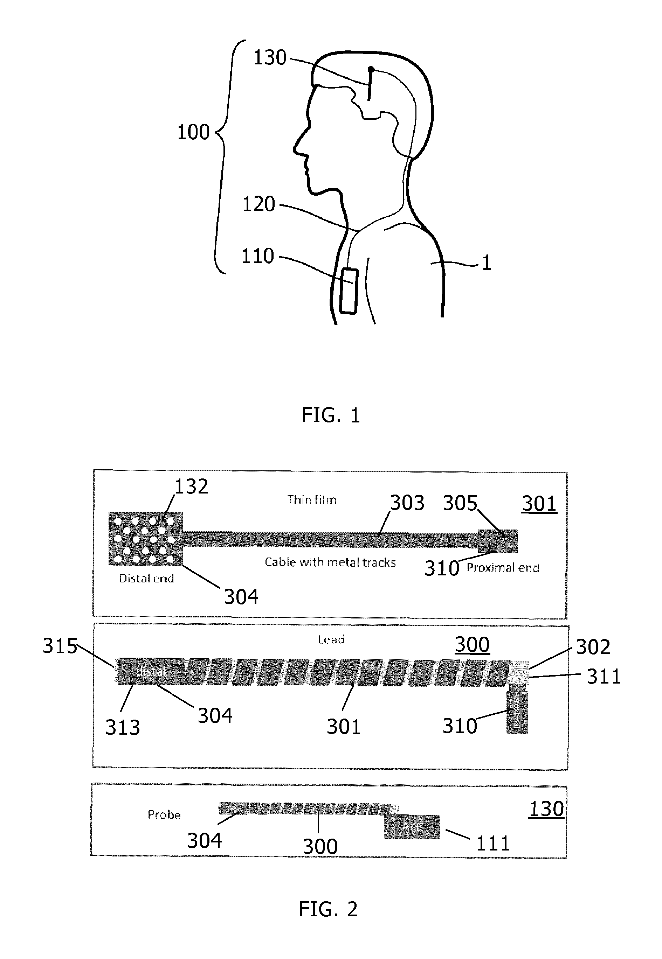

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an Advanced Lead Can element 111 comprising electronic means to address electrodes 132 on the distal end 304 of the thin film 301, which is arranged at the distal end 313 and next to the distal tip 315 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 arranged at the proximal end 311 of the lead 300 is electrically connected to the Advanced Lead Can element 111. The Advanced Lead Can element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises metal lines (not shown) to connect each distal electrodes 132 to a designated proximal contact 305.

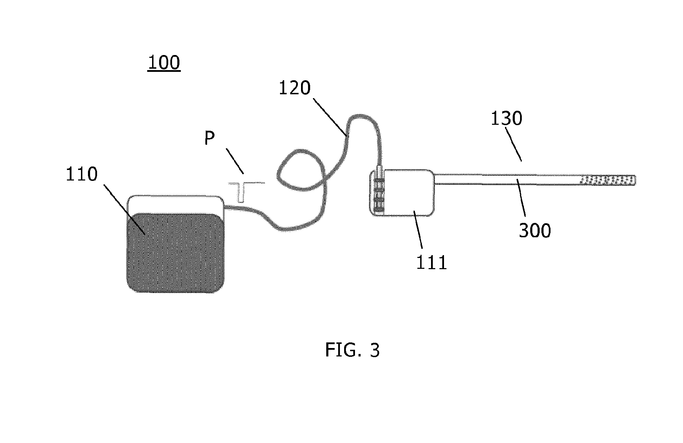

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the Advanced Lead Can 111. The controller 110 can be an implantable pulse generator (IPG) 110.

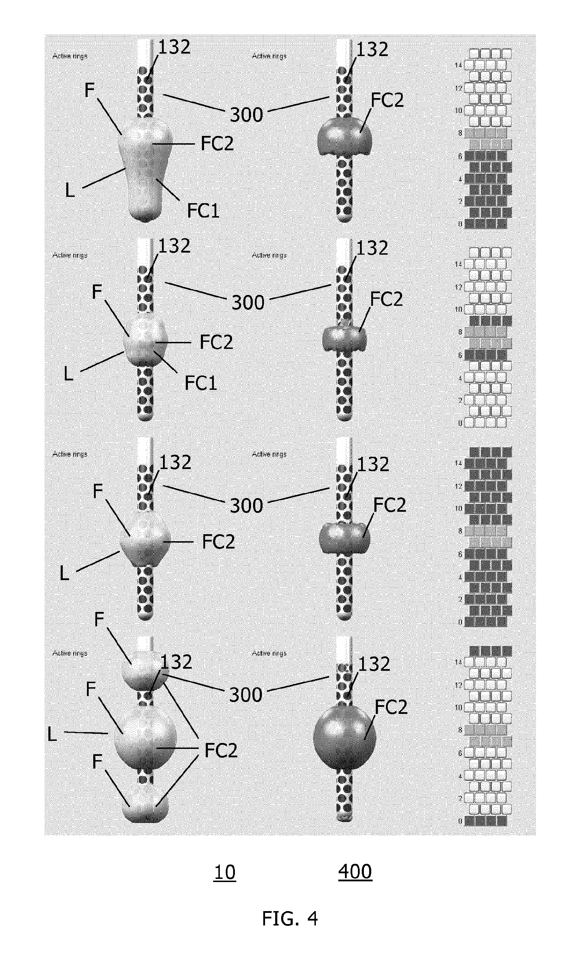

Figure 4 shows an overview of a possible embodiment of the system 10 according to the invention.

The system 10 is a system 10 for planning and/or providing a therapy for neural applications, in this embodiment a system 10 for neurostimulation and/or neurorecording applications, here for deep brain stimulation.

The lead 300 has a plurality of electrodes 132 which forms a complex geometrical array and/or that the plurality of electrodes 132 is arranged circumferentially around at least one section of the lead, especially around a section next to the distal tip end of the lead 300.

The processing means 400 is capable to calculate and to determine and to process characterizing data of a stimulation field being provided by the electrodes 132 comprising at least two different field components. The at least two different field components are a first field component FC1 having a first field vector and a second field component FC2 having a second field vector having a second field vector different from the first field vector. The processing means 400 is further configured such that the directional component of the stimulation field F may be determined.

The field vector may define the direction of the electric field, i.e. e.g. the stimulation field. The direction of the electric field may be defined by the force that is exerted on a positively charged particle.

Here, the first field component FC1 is an anodic field component and the second field component FC2 is a cathodic field component. The cathodic field component is provided by at least one (or more) electrode(s) 132 through which e.g. electric current flows out. The anodic field component is provided by at least one (or more) electrode(s) 132 through which e.g. electric current "flows in".

So, the first field component FC1 is a field component having a field vector pointing away from the lead 300 and the second field component FC2 is a field component having a field vector pointing towards from the lead 300.

The processing means 400 is configured such that the directional component of the stimulation field F is determined by determining and/or analysing the location L and the first and/or second field vector.

Two components of the location L and the first and/or second field vector are determined and are used for the determination of the directional component of the stimulation field F, especially that only the x-component and the y-component of the location L and the first and/or second field vector are determined and are used for the determination of the directional component of the stimulation field F.

The vectors and also the location, which can be considered also as a vector, may comprise a x-component, a y-component and a z-component. For instance, in a case, where the longitudinal axis of the lead is located along the z-axis, only the x-component and the y-component are to be considered.

By this, the advantage is achieved that the determination process is simplified and thus accelerated. Further, the determination process requires less calculation capability of the processing means and also less storage capability of the processing means. Additionally, by this the determination problem is reduced from a 3D-problem and 3D-calculation to a 2D-calculation.

Further, the processing means 400 is configured such that the dot product of the location L and the first and/or second field vector is determined, wherein especially it is determined that the first field component FC1 is a field component having a field vector pointing away from the lead 300 if the dot product is >0, i.e. has a value larger than zero, and the second field component (FC2) is a field component having a field vector pointing towards from the lead (300) is <0, i.e. has a value lower than zero.

For example, in order to define a stimulation field as anodic or cathodic, the stimulation field vectors together with their locations are considered. In the case when the stimulation field is an electric field F, assume we have an electric field vector, $\mathbf{E}$:

$$E = \begin{pmatrix} a \\ b \\ c \end{pmatrix}$$

The electric field F is located at coordinate (x, y, z) and the DBS lead 300 is located along the z-axis at (0, 0, z). The location L of the vector (x, y, z) may also be considered a vector. In order to decide if the electric field vector is pointing towards the lead 300 or away from the lead 300, the angle between the location vector and the vector itself is regarded. The dot product of two Euclidian vectors is directly related to the cosine of the angle between the vectors. In order to decide if a vector is pointing towards the lead or away from the lead we only need to consider the x and y components of the vectors, given that the lead is located along the z-axis. If the angle between the two vectors is smaller than $\pi/2$ radians (90 degrees) we know that the vector is pointing away from the lead and towards the lead otherwise. In our case it is not important to know the exact angle only that it is larger or smaller than 90 degrees. The dot product of two vectors are >0 if the angle is smaller than 90 degrees, and <0 if the angle is larger than 90 degrees. Thus, we defined the cathodic field as the vectors for which the dot product of the field vector and the coordinate vector is <0. In order to display the electric field with a 3D isosurface, or a colour-coded field map, the magnitude of each field vector must be calculated. This is done with Pythagoras theorem:

$$|a| = \sqrt{a_x{}^2 + a_y{}^2 + a_z{}^2}$$

Where $a$ is a vector and $a_i$ the different components of the vector.

An example of electric fields and cathodic fields produced by the same electrical settings are shown in Figure 4. The distribution of the stimulation fields as produced by a neurostimulator device 100 were visualized e.g. with a touch screen with electric fields (e.g. green) shown in the left column, cathodic fields (e.g. red) in the middle column, at an isolevel at 400 V/m. The electrical settings used to produce the fields were displayed with an unfolded contact array in the right column where purple refers to contacts that are grounded, and e.g. yellow displayed contacts that have a negative electric potential.

**Claims**

1. A system for planning and/or providing a therapy for neural applications, especially for neurostimulation and/or neurorecording applications, comprising at least one lead (300), the lead (300) having a plurality of electrodes (132), further comprising a processing means (400) being capable to calculate and/or to determine and/or to process characterizing data of a stimulation field (F) being provided by the electrodes (132), the characterizing data of the stimulation field (F) comprising at least two different field components (FC1; FC2), wherein the at least two different field components are a first field component (FC1) having a first field vector and a second field component (FC2) having a second field vector having a second field vector different from the first field vector, wherein the processing means (400) is further configured such that the directional component of the stimulation field (F) may be determined based on the characterizing data of the stimulation field (F).

2. The system (10) according to claim 1, characterized in that the plurality of electrodes (132) forms a complex geometrical array and/or that the plurality of electrodes (132) is arranged circumferentially around at least one section of the lead, especially around a section next to the distal tip end of the lead (300).

3. The system (10) according to claim 1 or 2, **characterized in that** the first field component (FC1) is an anodic field component and the second field component (FC2) is a cathodic field component.

4. The system (10) according to one of the preceding claims, **characterized in that** the first field component (FC1) is a field component having a field vector pointing away from the lead (300) and the second field component (FC2) is a field component having a field vector pointing towards from the lead (300).

5. The system (10) according to one of the preceding claims, **characterized in that** the processing means (400) is configured such that the directional component of the stimulation field (F) is determined directly and/or indirectly by determining and/or analysing the location (L) and the first and/or second field vector.

**6.** The system (10) according to according to claim 5, **characterized in that** the processing means (400) is configured such that two components of the location (L) and the first and/or second field vector are determined and are used for the determination of the directional component of the stimulation field (F), especially that only the x-component and the y-component of the location (L) and the first and/or second field vector are determined and are used for the determination of the directional component of the stimulation field (F).

**7.** The system (10) according to one of the preceding claims, **characterized in that** the processing means (400) is configured such that the dot product of the location (L) and the first and/or second field vector is determined, wherein especially it is determined that the first field component (FC1) is a field component having a field vector pointing away from the lead (300) if the dot product is >0, i.e. has a value larger than zero, and the second field component (FC2) is a field component having a field vector pointing towards from the lead (300) if the dot product is <0, i.e. has a value lower than zero.

**8.** A system for neural applications (100), especially a system for neurostimulation and/or neurorecording applications (100), for instance a deep brain stimulation system (100), the system for neural applications (100) comprising at least one system (10) for planning and/or providing a therapy for neural applications according to one of the preceding claims.

**9.** A method for determining the directional component of a stimulation field (F) provided by the electrodes (132) of a lead (300) based on characterizing data of the stimulation field (F), wherein the lead (300) is a lead (300) for neural applications, especially for neurostimulation and/or neurorecording applications, wherein the characterizing data of the stimulation field (F) comprising at least two different field components are calculated and/or determined and/or processed, wherein the at least two different field components are a first field component (FC1) having a first field vector and a second field component (FC2) having a second field vector having a second field vector different from the first field vector.

**10.** The method according to claim 9, characterized inthat the plurality of electrodes (132) forms a complex geometrical array and/or that the plurality of electrodes (132) is arranged circumferentially around at least one section of the lead, especially around a section next to the distal tip end of the lead (300).

**11.** The method according to claim 9 or 10,

**characterized** int hat the first field component (FC1) is an anodic field component and the second field component (FC2) is a cathodic field component and/or the first field component (FC1) is a field component having a field vector pointing away from the lead (300) and the second field component (FC2) is a field component having a field vector pointing towards from the lead (300).

**12.** The method according to one of claims 9 to 11, **characterized in that** the directional component of the stimulation field (F) is determined directly and/or indirectly by determining and/or analysing the location (L) and the first and/or second field vector.

**13.** The method according to one of claims 9 to 12, **characterized in that** two components of the location (L) and the first and/or second field vector are determined and are used for the determination of the directional component of the stimulation field (F), especially that only the x-component and the y-component of the location (L) and the first and/or second field vector are determined and are used for the determination of the directional component of the stimulation field (F).

**14.** The method according to one of claims 9 to 13, **characterized in that** the dot product of the location (L) and the first and/or second field vector is determined, wherein especially it is determined that the first field component (FC1) is a field component having a field vector pointing away from the lead (300) if the dot product is >0, i.e. has a value larger than zero, and the second field component (FC2) is a field component having a field vector pointing towards from the lead (300) if the dot product is <0, i.e. has a value lower than zero.

**15.** The method according to one of claims 9 to 14, **characterized in that** the method is conducted with at least one system (10) for planning and/or providing a therapy for neural applications according to one of claims 1 to 7 and/or a system for neural applications (100) according to claim 8.

FIG. 1

FIG. 2

100

120

P

110

130

111

300

FIG. 3

10                    400

FIG. 4

Europäisches
Patentamt

European
Patent Office

Office européen
des brevets

## EUROPEAN SEARCH REPORT

Application Number

EP 13 17 0846

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/165898 A1 (MOFFITT MICHAEL A [US]) 28 June 2012 (2012-06-28) * abstract; figures 1-9 * * paragraph [0002] * * paragraph [0016] - paragraph [0018] * * paragraph [0027] - paragraph [0037] * * paragraph [0039] - paragraph [0055] * * paragraph [0066] - paragraph [0086] * ----- | 1-15 | INV. A61N1/36 ADD. A61N1/372 G06F19/00 |
| A | US 2011/191275 A1 (LUJAN J LUIS [US] ET AL) 4 August 2011 (2011-08-04) * the whole document * ----- | 1-15 | |
| A | WO 2011/098937 A1 (KONINKL PHILIPS ELECTRONICS NV [NL]; MARTENS HUBERT CECILE FRANCOIS [N) 18 August 2011 (2011-08-18) * the whole document * ----- | 1-15 | |
| A | WO 2009/139917 A2 (INTELECT MEDICAL INC [US]; SPARKS TROY [US]; BARNHORST JORDAN [US]; BL) 19 November 2009 (2009-11-19) * the whole document * ----- | 1-15 | TECHNICAL FIELDS SEARCHED (IPC) A61N G06F |

The present search report has been drawn up for all claims

| Place of search | Date of completion of the search | Examiner |
|---|---|---|
| Munich | 10 October 2013 | Molina Silvestre, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 13 17 0846

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2013

| Patent document cited in search report | | | Publication date | Patent family member(s) | | | Publication date |
|---|---|---|---|---|---|---|---|
| US 2012165898 | A1 | | 28-06-2012 | AU | 2011352302 | A1 | 11-07-2013 |
| | | | | CA | 2823047 | A1 | 05-07-2012 |
| | | | | EP | 2658606 | A1 | 06-11-2013 |
| | | | | US | 2012165898 | A1 | 28-06-2012 |
| | | | | WO | 2012092206 | A1 | 05-07-2012 |
| US 2011191275 | A1 | | 04-08-2011 | AU | 2010286603 | A1 | 22-03-2012 |
| | | | | CA | 2772330 | A1 | 03-03-2011 |
| | | | | EP | 2470258 | A1 | 04-07-2012 |
| | | | | JP | 2013502999 | A | 31-01-2013 |
| | | | | US | 2011191275 | A1 | 04-08-2011 |
| | | | | US | 2013218819 | A1 | 22-08-2013 |
| | | | | WO | 2011025865 | A1 | 03-03-2011 |
| WO 2011098937 | A1 | | 18-08-2011 | CN | 102762253 | A | 31-10-2012 |
| | | | | EP | 2533851 | A1 | 19-12-2012 |
| | | | | JP | 2013519418 | A | 30-05-2013 |
| | | | | KR | 20130008534 | A | 22-01-2013 |
| | | | | US | 2012303089 | A1 | 29-11-2012 |
| | | | | WO | 2011098937 | A1 | 18-08-2011 |
| WO 2009139917 | A2 | | 19-11-2009 | EP | 2321001 | A1 | 18-05-2011 |
| | | | | EP | 2321002 | A1 | 18-05-2011 |
| | | | | EP | 2321003 | A1 | 18-05-2011 |
| | | | | EP | 2321004 | A2 | 18-05-2011 |
| | | | | EP | 2321005 | A1 | 18-05-2011 |
| | | | | US | 2009287271 | A1 | 19-11-2009 |
| | | | | US | 2009287272 | A1 | 19-11-2009 |
| | | | | US | 2009287273 | A1 | 19-11-2009 |
| | | | | US | 2009287467 | A1 | 19-11-2009 |
| | | | | US | 2010049276 | A1 | 25-02-2010 |
| | | | | US | 2011313268 | A1 | 22-12-2011 |
| | | | | US | 2011313487 | A1 | 22-12-2011 |
| | | | | US | 2012265267 | A1 | 18-10-2012 |
| | | | | US | 2012271376 | A1 | 25-10-2012 |
| | | | | US | 2013226261 | A1 | 29-08-2013 |
| | | | | WO | 2009139892 | A1 | 19-11-2009 |
| | | | | WO | 2009139907 | A1 | 19-11-2009 |
| | | | | WO | 2009139909 | A1 | 19-11-2009 |
| | | | | WO | 2009139910 | A1 | 19-11-2009 |
| | | | | WO | 2009139917 | A2 | 19-11-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010055453 A1

- US 7941202 B